Europäisches Patentamt

European Patent Office

Office européen des brevets

(11) Numéro de publication: **0 336 853**
**A1**

## (12) DEMANDE DE BREVET EUROPEEN

(21) Numéro de dépôt: 89420034.4

(22) Date de dépôt: 03.02.89

(51) Int. Cl.⁴: **A 61 M 37/00**

(30) Priorité: 07.04.88 FR 8804974

(43) Date de publication de la demande:
11.10.89 Bulletin 89/41

(84) Etats contractants désignés:
AT BE CH DE ES FR GB GR IT LI LU NL SE

(71) Demandeur: **CAIR (SOCIETE ANONYME DE DROIT FRANCAIS)**
**4-6, rue de l'Union**
**F-69170 Tarare (FR)**

(72) Inventeur: **Lopez, Georges Antoine**
**Rue des Phily**
**F-69290 CRAPONNE (FR)**

(74) Mandataire: **Maureau, Philippe et al**
**Cabinet GERMAIN & MAUREAU BP 3011**
**F-69392 Lyon Cédex 03 (FR)**

(54) Boîtier de protection d'un site de connexion à usage médical.

(57) Ce boîtier est du type comprenant deux demi-coquilles (3,4) en matière synthétique venant ensemble de moulage et articulées l'une sur l'autre le long d'une ligne (5) correspondant à un amincissement de matière et comportant, sur d'autres faces que celle présentant l'articulation, des moyens de fermeture élastique, les deux demi-coquilles étant chacune garnies de mousse (12) destinée à être imbibée d'agent antiseptique, et comportant des empreintes (14) pour l'encastrement d'un robinet (6) ou d'une rampe de robinets, les parois du boîtier comportant des découpes complémentaires (10) destinées, en position fermée du boîtier, à délimiter des ouvertures pour le passage des tubulures reliées au site.

Selon l'invention la demi-coquille (3) constitutive du fond est équipée de moyens (13) de fixation amovible du site de connexion.

FIG. 3

EP 0 336 853 A1

## Description

### Boîtier de protection d'un site de connexion à usage médical

La présente invention a pour objet un boîtier de protection d'un site de connexion à usage médical.

De nombreux liquides médicaux sont administrés aux patients par voie parentérale, qu'il s'agisse de médicaments ou de sérums d'alimentation. Le patient est généralement équipé d'un cathéter engagé dans une veine, dans lequel débouche une tubulure d'amenée des liquides. Afin d'adapter le traitement à l'état instantané du patient, il est nécessaire de pouvoir injecter simultanément ou en alternance plusieurs liquides. A cet effet, la ligne est équipée, à son extrémité opposée à celle comportant le cathéter, d'un site de connexion de plusieurs tubulures comportant un robinet à trois voies ou une rampe de robinets.

Afin d'éviter les risques de contamination du patient il est nécessaire de maintenir ce site en conditions stériles.

Une première solution consiste à envelopper ce site dans un pansement imbibé d'agent antiseptique. Cette solution n'est pas satisfaisante, puisque ce pansement est salissant tant pour le patient que pour le personnel médical, et qu'il doit être retiré pour pratiquer toute manoeuvre sur le site, qu'il s'agisse de la réalisation d'une connexion ou de la manoeuvre du robinet.

Il a donc été imaginé de réaliser une protection d'un site à l'aide d'un boîtier comprenant deux demi-coquilles en matière synthétique venant ensemble de moulage et articulées l'une sur l'autre le long d'une ligne correspondant à un amincissement de matière. Les deux demi-coquilles sont garnies chacune de mousse comportant des empreintes pour l'encastrement du site, cette mousse étant destinée à être imbibée par un agent antiseptique. Les parois du boîtier présentent des découpes complémentaires délimitant des ouvertures pour le passage des tubulures, et sont équipées de moyens de fermeture élastiques. Généralement la fermeture est réalisée par accrochage élastique d'éléments disposés au niveau des deux parois latérales du boîtier, c'est-à-dire des deux parois adjacentes à la paroi équipée de l'articulation.

Il en résulte que l'opérateur doit utiliser ses deux mains pour réaliser une ouverture du boîtier. Or le site n'étant pas maintenu fermement dans le boîtier risque au cours de cette opération de s'échapper de celui-ci. En outre, toute manoeuvre du boisseau du robinet nécessite que l'utilisateur maintienne le robinet plaqué à l'aide d'au moins un doigt d'une main dans le fond du boîtier. Or ce contact nuit à la stérilité du site.

D'autre part, pour réaliser une connexion sur une tubulure transversale à la tubulure principale, il est nécessaire de retirer totalement le site hors du boîtier, ce qui est une opération nuisant à la stérilité. Enfin, un tel boîtier ne permet pas de visualiser la position du boisseau d'un robinet ni d'éventuelles fuites de liquide au niveau de ce robinet sans ouverture du boîtier.

La présente invention vise à remédier à ces inconvénients.

A cet effet, dans le boîtier qu'elle concerne, qui est du type précité, la demi-coquille constitutive du fond est équipée de moyens de fixation amovible du site de connexion. Il en résulte que lors de l'ouverture du boîtier, il n'y a pas de risque de désolidarisation du site et du boîtier, et qu'il est possible de manoeuvrer le boisseau d'un robinet sans avoir à maintenir le site de connexion dans le boîtier, ce qui favorise les conditions d'asepsie.

Conformément à une possibilité, les moyens de fixation du site sont des moyens de fixation par encliquetage élastique. Il est ainsi possible de réaliser très facilement le montage et le démontage du site tout en assurant une bonne tenue de celui-ci dans le fond du boîtier lorsque cela est souhaité.

Avantageusement, dans ce boîtier destiné au logement d'un site de connexion comportant une tubulure principale de passage de fluide dans laquelle débouche radialement au moins une tubulure qui en est perpendiculaire chacune au niveau d'un robinet, les moyens de fixation du site sont constitués par des pièces faisant saillie du fond, en forme générale de demi-anneaux dont l'ouverture est rétrécie, alignés selon l'axe de la tubulure principale et destinées à permettre l'encliquetage élastique de celle-ci, avec possibilité de rotation autour de son axe, en position encliquetée.

Ce montage est particulièrement intéressant puisque permettant, sans désolidarisation du site et du boîtier, de faire pivoter le site par rapport au boîtier, par exemple en exerçant une action sur le levier de manoeuvre du boisseau. permettant ainsi de dégager hors de la mousse le tronçon de tubulure perpendiculaire à la tubulure principale, pour réaliser une injection ou pour le connecter à une tubulure.

Selon une astre caractéristique de l'invention le boîtier est réalisé en une matière synthétique translucide, la paroi supérieure du couvercle comportant en regard de chaque robinet une surface polie, la mousse présentant elle-même une ouverture entre cette surface polie et le robinet.

Le fait que le boîtier soit réalisé en matière translucide permet de déceler, sans avoir à l'ouvrir, les éventuelles fuites de liquide qui pourraient intervenir par exemple au niveau du boisseau du robinet.

La surface polie du couvercle du boîtier permet pour sa part de visualiser instantanément, sans ouverture du boîtier la position du robinet, ce qui favorise les conditions de protection du site, en limitant le nombre d'ouvertures du boîtier.

Conformément à une autre caractéristique de l'invention, ce boîtier est équipé de moyens de fermeture disposés sur sa paroi avant, c'est-à-dire celle opposée à la paroi comportant l'articulation. Selon une possibilité les moyens de fermeture sont constitués par une zone d'épaisseur augmentée de matière sur le fond et une zone d'épaisseur augmentée de matière sur le couvercle, comportant des organes d'accrochage complémentaires. Pour réaliser l'ouverture du boîtier, il suffit à l'opérateur tenant le boîtier dans une main d'exercer une pression sur la paroi avant du boîtier, dans la zone comportant une surépaisseur pour libérer les moyens d'accrochage. Le fait que l'ouverture soit réalisée à l'aide d'une seule main permet à l'opérateur de manipuler le boîtier et éventuellement le boisseau du robinet sans avoir à changer de prise.

De toute façon, l'invention sera bien comprise à l'aide de la description qui suit, en référence au dessin schématique annexé, représentant à titre d'exemple non limitatif une forme d'exécution de ce boîtier :

Figure 1 en est une vue en perspective en position fermée ;

Figure 2 en est une vue en perspective en position ouverte ;

Figure 3 est une vue en perspective similaire à celle de figure 2, le site de connexion étant retiré ;

Figure 4 en est une vue en coupe transversale, en position ouverte, le site de connexion etant en position basculée.

Le boîtier représenté au dessin, désigné par la référence générale 2, comprend deux demi-coquilles 3 et 4, dont celle 3 est constitutive du fond et dont celle 4 est constitutive du couvercle. Ces deux demi-coquilles 3 et 4 viennent de moulage en une seule pièce et sont reliées l'une à l'autre par une zone amincie 5 de matière formant une charnière-film. Ce boîtier 2 est destiné à recevoir un site de connexion 6 comprenant une tubulure principale 7 traversant en ligne droite le corps 8 d'un robinet dans lequel débouche radialement une tubulure 9. Ce robinet à trois voies est actionné par un boisseau dont le levier porte la référence 11. Le fond 3 et le couvercle 4 comportent, dans leurs parois latérales et avant, des découpes complémentaires 10 assurant, en position de fermeture, le ménagement d'ouvertures pour le passage des tubulures raccordées au site 6.

De façon connue en soi, les deux demi-coquilles 3 et 4 sont garnies de mousse éponge 12.

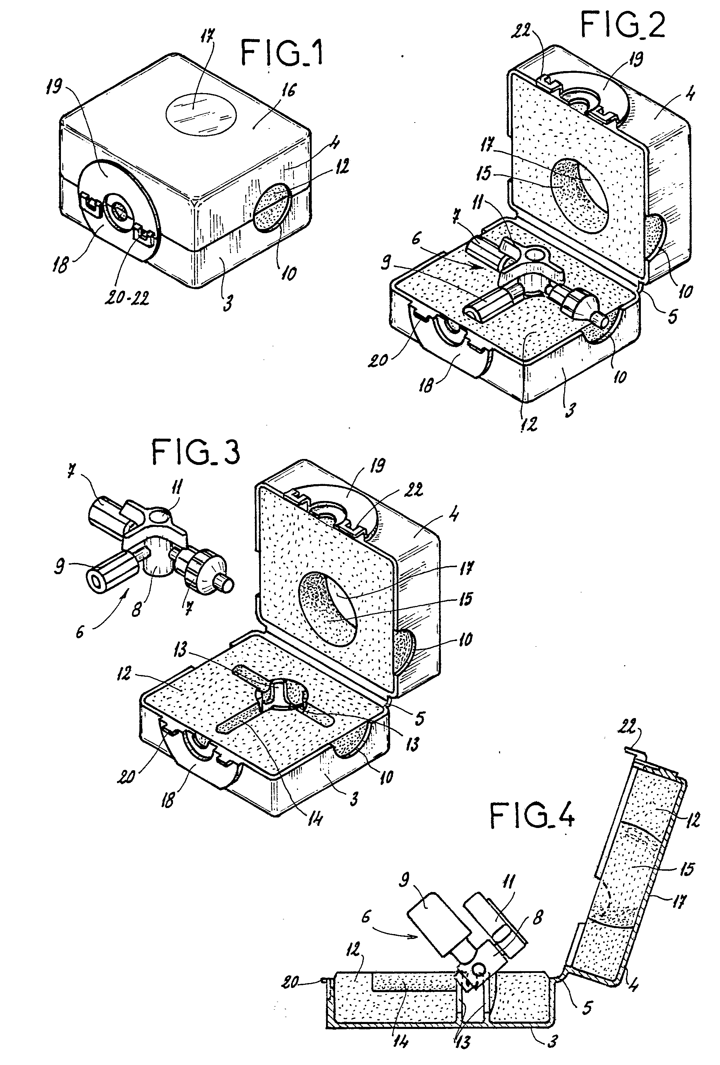

Comme montré à la figure 3, du fond 3 du boîtier fait saillie un dispositif d'accrochage du site comprenant deux demi-anneaux 13 ouverts vers le haut. Ces deux demi-anneaux de diamètre correspondant à celui de la tubulure 7 comportent une ouverture rétrécie.

Dans la forme d'exécution représentée au dessin ces deux demi-anneaux sont ménagés dans une même cheminée dont les parties avant et arrière sont évidées. La mousse 12 logée dans le fond 3 du boîtier comporte une empreinte 14 pour l'encastrement du site 6. Ce dernier peut donc être fixé dans les anneaux 13 par encliquetage élastique. Le fait que cette fixation soit réalisée par des pièces en forme d'anneau permet, comme cela est représenté à la figure 4, de faire basculer le robinet vers l'arrière, sans avoir à le désolidariser du boîtier, par exemple en exerçant une pression sur le seul levier de manoeuvre 11, par exemple lorsqu'il est nécessaire de réaliser une connexion sur l'embout 9. La mousse 12 logée dans le couvercle 4 présente en regard du levier de manoeuvre 11 une cheminée 15 s'étendant jusqu'à la paroi supérieure 16. Dans cette paroi 16 est ménagée, en regard de la cheminée 15, une surface polie 17 formant une fenêtre permettant une visualisation immédiate, de la position du boisseau du robinet sans avoir à ouvrir le boîtier.

Sur sa face avant, c'est-à-dire celle opposée à la charnière 5, le boîtier présente deux zones, respectivement 18 sur le fond et 19 sur le couvercle, dont l'épaisseur est renforcée. Les zones 18 et 19 sont équipées respectivement de doigts 20 et de crochets 22 permettant un accrochage élastique. Pour réaliser l'ouverture du boîtier, il suffit à l'opérateur tenant celui-ci dans une main, d'exercer une simple pression sur la zone 15, ce qui permet l'escamotage des doigts 20 hors des crochets 22.

Comme il ressort de ce qui précède, l'invention apporte une grande amélioration à la technique existante, en fournissant un boîtier de protection d'un site de connexion, de conception simple d'un maniement très pratique pour le personnel hospitalier et assurant un très bon maintien dans des conditions aseptiques, en limitant au maximum les nécessités d'ouverture du boîtier ainsi que les contacts entre le site lui-même et les doigts d'un opérateur.

## Revendications

1. Boîtier de protection d'un site de connexion à usage médical, du type comprenant deux demi-coquilles (3,4) en matière synthétique venant ensemble de moulage et articulées l'une sur l'autre le long d'une ligne (5) correspondant à un amincissement de matière et comportant, sur d'autres faces que celle présentant l'articulation, des moyens de fermeture élastique, les deux demi-coquilles étant chacune garnies de mousse (12) destinée à être imbibée d'agent antiseptique, et comportant des empreintes (14) pour l'encastrement d'un robinet (6) ou d'une rampe de robinets, les parois du boîtier comportant des découpes complémentaires (10) destinées, en position fermée du boîtier, à délimiter des ouvertures pour le passage des tubulures reliées au site, caractérisé en ce que la demi-coquille (3) constitutive du fond est équipée de moyens (13) de fixation amovible du site de connexion.

2. Boîtier selon la revendication 1, caractérisé en ce que les moyens (13) de fixation du site sur le fond, sont des moyens de fixation par encliquetage élastique.

3. Boîtier selon l'une quelconque des revendications 1 et 2, destiné au logement d'un site de connexion comportant une tubulure principale (7) de passage de fluide dans laquelle débouche radialement au moins une tubulure (9) qui en est perpendiculaire chacune au niveau d'un robinet, caractérisé en ce que les moyens de fixation du site sont constitués par des pièces (13) faisant saillie du fond, en forme générale de demi-anneaux dont l'ouverture est rétrécie, alignés selon l'axe de la tubulure principale (7), et destinées à permettre l'encliquetage élastique de celle-ci, avec possibilité de rotation autour de son axe, en position encliquetée.

4. Boîtier selon l'une quelconque des revendications 1 à 3, caractérisé en ce que le boîtier est réalisé en une matière synthétique translucide, la paroi supérieure du couvercle comportant en regard de chaque robinet (6) une surface polie (17), la mousse présentant elle-même une ouverture (15) entre cette surface polie (17) et le robinet (6).

5. Boîtier selon l'une quelconque des revendications 1 à 4, caractérisé en ce qu'il est équipé de moyens de fermeture disposés sur sa paroi avant, c'est-à-dire celle opposée à la paroi comportant l'articulation.

6. Boîtier selon la revendication 5, caractérisé en ce que les moyens de fermeture sont constitués par une zone (18) d'épaisseur augmentée de matière sur le fond et une zone (19) d'épaisseur augmentée de matière sur le couvercle, comportant des organes d'accrochage complémentaires (20,22).

FIG_1

FIG_2

FIG_3

FIG_4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 578 746 (SIFIC)<br>* Revendications 1-2,4; figure 2 *<br>--- | 1-3,5-6 | A 61 M 37/00 |
| Y | CH-A- 634 902 (VIFOR S.A.)<br>* Figure 1; page 2, colonne de droite, lignes 35-39 *<br>--- | 1-3,5-6 | |
| Y | US-A-2 601 101 (DERHAM)<br>* Figures 1-2 *<br>--- | 6 | |
| A | GB-A-2 127 380 (PEAK PLASTIC & METAL PRODUCTS LTD)<br>* Page 2, lignes 65-80 *<br>----- | 4 | |

DOMAINES TECHNIQUES RECHERCHES (Int. Cl.4)

A 61 M
B 65 D
A 65 D

Le présent rapport a été établi pour toutes les revendications

| Lieu de la recherche | Date d'achèvement de la recherche | Examinateur |
|---|---|---|
| LA HAYE | 24-04-1989 | MIR Y GUILLEN V. |

EPO FORM 1503 03.82 (P0402)